(19) Europäisches Patentamt
European Patent Office
Office européen des brevets

(11) EP 0 751 958 B1

(12) **EUROPEAN PATENT SPECIFICATION**

(45) Date of publication and mention of the grant of the patent:
**09.11.2005 Bulletin 2005/45**

(21) Application number: **95911477.8**

(22) Date of filing: **24.03.1995**

(51) Int Cl.[7]: **C07K 14/435**, C08B 37/00, A61K 38/17, A61K 31/725, G01N 33/50

(86) International application number:
**PCT/IB1995/000208**

(87) International publication number:
**WO 1995/025745 (28.09.1995 Gazette 1995/41)**

(54) **FLUCOSE CONTAINING PROTEOGLYCAN OR ACID GLYCAN AND THEIR PHARMACEUTICAL USE**

FUCOSE ENTHALTENDES PROTEOGLYCAN ODER SAURES GLYCAN UND SEINE PHARMAZEUTISCHE VERWENDUNG

PROTEOGLYCANES OU GLYCANES ACIDES CONTENANT DU FUCOSE ET LEUR UTILISATION PHARMACEUTIQUE

(84) Designated Contracting States:
**CH DE FR GB LI**

(30) Priority: **24.03.1994 GB 9405846**

(43) Date of publication of application:
**08.01.1997 Bulletin 1997/02**

(73) Proprietor: **Misevic, Gradimir**
**4125 Riehen (CH)**

(72) Inventor: **Misevic, Gradimir**
**4125 Riehen (CH)**

(56) References cited:


- **JOURNAL OF BIOLOGICAL CHEMISTRY, vol.262, no.12, 25 April 1987, BALTIMORE, MD US pages 5870 - 5877 G. MISEVIC ET AL. 'INVOLVEMENT OF CARBOHYDRATES AS MULTIPLE LOW AFFINITY INTERACTION SITES IN THE SELF-ASSOCIATION OF THE AGGREGATION FACTOR FROM THE MARINE SPONGE MICROCIONA PROLIFERA.' cited in the application**
- **JOURNAL OF BIOLOGICAL CHEMISTRY, vol.265, no.33, 25 November 1990, BALTIMORE, MD US pages 20577 - 20584 G. MISEVIC ET AL. 'THE SPECIES-SPECIFIC CELL-BINDING SITE OF THE AGGREGATION FACTOR FROM THE SPONGE MICROCIONA PROLIFERA IS A HIGHLY REPETITIVE NOVEL GLYCAN CONTAINING GLUCURONIC ACID, FUCOSE, AND MANNOSE.' cited in the application**
- **JOURNAL OF BIOLOGICAL CHEMISTRY, vol.268, no.7, 5 March 1993, BALTIMORE, MD US pages 4922 - 4929 G. MISEVIC ET AL. 'CARBOHYDRATE-CARBOHYDRATE INTERACTIONS OF A NOVEL ACIDIC GLYCAN CAN MEDIATE SPONGE CELL ADHESION.' cited in the application**
- **JOURNAL OF CELLULAR BIOCHEMISTRY, vol.53, no.2, October 1993, NEW YORK, N.Y., US pages 98 - 113 E. PAPAKONSTANTINOU ET AL. 'ISOLATION AND CHARACTERIZATION OF A NEW CLASS OF ACIDIC GLYCANS IMPLICATED IN SEA URCHIN EMBRYONAL CELL ADHESION.'**


Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### TECHNICAL FIELD :

This invention relates to a class of proteoglycans having fucosylated acidic glycan side chains bound to a protein backbone which have been found to stimulate selectively proliferation of natural killer (NK) cells and/or γδT cells. They are useful as immunostimulants, e.g., in the treatment of cancer and viral infections.

### PRIOR ART :

The proteoglycans referred to in the invention are produced by proliferating cells, for example by sponge cells, sea urchin cells, and, in the case of higher animals (including humans), by embryonic cells and tumor cells. In the natural proteoglycan form, the compounds are large (ca. 5000 to 30,000 kD) extracellular or membrane-bound molecules having a protein backbone which is glycosylated with acidic glycan chains having an unusual polysaccaride sequence containing internal fucose. The structure of the acidic glycan side chains of the proteoglycan isolated from the marine sponge *Microciona prolifera* has been partially characterized (Spillmann, et al., J. Biol. Chem (1993) 268: 13378-13387), and we have previously shown that this proteoglycan is involved in cellular aggregation (Misevic, et al., J. Biol. Chem. (1987) 262: 5870-5877; Misevic, et al., J. Biol. Chem. (1990) 265: 20577-20584 ; Misevic, et al., J. Biol. Chem. (1993) 268: 4922-4929). A sulfated blood group Lewis[a], having a fucose, is disclosed in J. Biol. Chem. 269 (Jan 1994) 1595 - 8 (Guen et al.). The previously undescribed protein backbone of the *Microciona prolifera* proteoglycan has now been isolated and characterized, and proteoglycans derived from sponges of other genera have also been characterized, as described below.

BRIEF DESCRIPTION OF THE INVENTION:

It has now surprisingly been discovered that these compounds are potent stimulators of NK cells and γδT cells. In particular, compounds isolated are:

- from organisms of the Phylum Porifera e.g., of the class Demospongiae, especially of the order Poecilosclerida, family Microcionidae (e.g., of the genus *Microciona*), or family Mycalidae (e.g., of the genus *Mycale*), or the order Halichondrida, family Halichondridae (e.g., of the genus *Halichondria*), or the order Hadromerida, family Clionidae (e.g., of the genus *Cliona*), or the order Haplosclerida, family Haliclonidae (e.g., of the genus *Haliclona*),
- and/or from organisms of the phylum Echinodermata.

These compounds have been shown to stimulate selectively different clones of NK cells and γδT cells. Moreover, it has been found that these compounds have significant anticancer, especially antimetastatic, effects *in vivo.* It is believed that these anticancer effects are due to stimulation *in vivo* of NK cells and/or γδT cells. The precise mechanism of this stimulation is unclear, but without intending to be bound by a particular theory, we suggest that these cells may be stimulated by polyvalent interactions with fucosylated acidic glycans of the class described herein and in this way can identify and destroy hyperproliferating cells expressing similar glycan structures. In a pathogenic case, where the hyperproliferating cells are not destroyed in this manner, it is believed that although the hyperproliferating cells produce these acidic glycans, they shed them or present them in monovalent form or other nonstimulatory or inhibitory form, thereby evading detection and destruction by NK cells and/or γδT cells specific for such acidic glycans. Application of the compounds of the invention stimulates NK cells and/or γδT cells specific for such cancer cells, thereby leading to their destruction. Additionally, the compounds of the invention are useful for stimulating NK cells and/or γδT cells against viral or retroviral infections. Finally, in monovalent form, the compounds of the invention are useful for inhibiting the activation of NK cells and/or γδT cells, thereby finding utility as immunosuppressants.

The compounds used in the invention are selective in their action, in that particular compounds of the invention stimulate only particular clones or subpopulations of NK cells or γδT cells. No significant stimulation of B cells or αβ T cells is observed, so undesirable immunostimulation, e.g., an allergenic or autoimmune response, is avoided. Despite this selectivity, all humans tested, from a variety of ethnic and racial groups, have cell populations capable of being significantly stimulated by the compounds of the invention. Compounds having the glycan structures of the class described herein are found in a wide variety of hyperproliferating cells from sponges to human tumors, thus the basic structure of the compounds is highly conserved. It is hypothesized that compounds of the class described herein act as signals for stimulating the body's defenses against unwanted proliferation of cancerous or infected cells, and that cancers or resistant viral infections may arise when, as described above, these compounds are secreted in nonstimulatory form. Among the examples described herein, it is noted that compounds used in the invention isolated from those of the genus *Microciona* are more effective in stimulating NK cells, as described in example 1 below, whereas

compounds isolated from the genus *Halichondria* are more effective in stimulating γδ T cells, as described in example 9, thus selectivity among cell types receptive to this stimulation is also possible.

## DETAILED DESCRIPTION OF THE INVENTION

The invention thus provides

1. Fucose-containing proteoglycans and acidic glycans thereof, preferably proteoglycans, isolated or capable of being isolated from an organism of the phylum Porifera, e.g., as described above, especially of the genera *Microciona* and/or *Halichondria* and/or *Mycale* and/or *Cliona* and/or from an organism of the phylum Echinodermata especially of the genus *Lytechinus* for use as a pharmaceutical or therapeutic agent; and pharmaceutical compositions comprising such compounds in combination with a pharmaceutically acceptable carrier or diluent.

2. Fucose-containing proteoglycans and acidic glycans thereof preferably proteoglycans, isolated or capable of being isolated from organisms of the genus *Halichondria* and/or *Mycale* and/or *Cliona.*

3. Fucose-containing acidic glycans capable of being isolated from a sea urchin of the genus *Lytechinus.*

4. A method of stimulating the proliferation of mammalian, e.g., human, NK cells and/or γδT cells comprising contacting said cells with a compound used in the invention (a fucose-containing proteoglycan and acidic glycan thereof, preferably a proteoglycan, isolated or capable of being isolated from an organism of the phylum Porifera, or Echinodermata e.g., as described above, especially of the genera *Microciona* and/or *Halichondria* and/or *Mycale* and/or *Cliona,* and/or of the phylum Echinodermata especially of the genus *Lytechinus,* in an *ex vivo* setting or *in vivo,* e.g., as a vaccine; or a method of treating cancer (e.g., preventing or inhibiting onset, growth, or metastasis of a tumor), or treating or preventing a viral or retroviral infection, in a mammal, comprising administering a pharmaceutically effective amount of a compound used in the invention to a patient in need of such treatment; or the use of said a compound in the manufacture of a medicament for treatment or prevention of cancer or viral or retroviral infections.

5. The use of a fucose-containing proteoglycan or acidic glycan thereof, preferably a proteoglycan, isolated or capable of being isolated from an organism of the phylum Porifera and/or Echinodermata, e.g., as described above, especially of the genera *Microciona* and/or *Halichondria,* and/or *Mycale* and/or *Cliona* for the phylum Porifera, especially of the genus *Lytechinus* for the phylum Echinodermata for *ex vivo* stimulation of proliferation of NK cells and/or γδT cells.

6. A method for screening for or detecting an immunosuppressive (e.g., NK cell and/or γδT cell inhibitory) compound comprising measuring proliferation of NK cells and/or γδT cells in a system containing an NK cell or γδT cell stimulatory concentration of a compound used in the invention in the presence and absence of a test compound.

Appropriate dosages of the compounds will of course vary, e.g. depending on the condition to be treated (for example the disease type or the nature of resistance), the effect desired, and the mode of administration. In general however satisfactory results are obtained on administration orally, rectally, nasally, topically, or parenterally, e.g. intravenously, for example by i.v. drip or infusion, at dosages on the order of from 0.01 to 2.5 up to 5 mg/kg, e.g. on the order of from 0.05 or 0.1 up to 1.0 mg/kg. Suitable dosages for patients are thus on the order of from 0.5 to 125 up to 250 mg i.v., e.g. on the order of from 2.5 to 50 mg i.v.. Pharmaceutical compositions of the invention may be manufactured in conventional manner, in a suitable aqueous carrier, for example sterile buffered physiological saline.

For *ex vivo* stimulation of cells, as described more fully in the example below, a suitable amount, e.g., at least 10 ml, of the patient's blood is removed, peripheral blood mononuclear cells are isolated from the blood, placed in a complete medium in the presence of a stimulatory concentration of a compound of the invention, e.g., 10-500 μg/ml, ca. 100 μg/ml, optionally in the presence of IL-2, and the culture is maintained until a significant increase in the population of the desired cell type is observed, e.g., ca. 2-4 weeks. Following stimulation of the cells, the cells are isolated from the medium, placed in an injection solution, e.g., sterile buffered physiological saline or plasma, and injected back into the patient. The compound of the invention for this use can, for example, be a proteoglycan or acidic glycan derived from a marine sponge as described in the examples, but may also be a proteoglycan, acidic glycan or fragment thereof isolated from a culture of the cancerous cells to be treated.

**INDUSTRIAL APPLICATION:**

The compounds can be useful notably as pharmaceuticals, particularly as immunostimulants, e.g. in the treatment of cancer and viral infections.

**EXAMPLES:**

**EXAMPLE 1: Preparation of proteoglycan and acidic glycans from *Microciona prolifera***

a. Extraction of proteoglycan from *Microciona prolifera.*

Fresh marine sponges (*Microciona prolifera*) collected from the Cape Cod area (USA) are rinsed with 0.5M NaCl, 0.18g/l $NaHCO_3$ (buffer A) and cut into cubes 1x1 cm. The cubes are incubated in the buffer A (50% suspension) for 12h at +4°C under gentle rotation. After filtration of the sponge cubes suspension through cheese cloth, the cubes were two more times extracted with the buffer A using the same incubation conditions. The supernatants are either combined or separately centrifuged at 3000 x g for 30 min at +4°C, and the obtained supernatant is again centrifuged at 12,000 x g for 40 min at +4°C. $CaCl_2$ is added to the supernatant to a concentration of 20mM. After 2-12 h gentle shaking at room temperature, the precipitated proteoglycan is centrifuged at 3000 x g for 20 min at room temperature. The pelleted proteoglycan is dissolved in at lest 20 volumes of 0.5 M NaCl, 2mM $CaCl_2$, 20mM Tris pH 7.4 (buffer B) and centrifuged at 10,000 x g for 30 min at +4°C to remove insoluble material. Supernatant was centrifuged at 100,000 x g for 4h at +4°C, and the pelleted proteoglycan redissolved in buffer B at concentration of 1-2 mg/ml. To the dissolved proteoglycan in buffer B solid CsCl is added to make a 50% concentration, and the solution is centrifuged in a SW rotor at 100,000 x g for 36h at room temperature. The pelleted proteoglycan is dialyzed against buffer B and stored at +4°C in the presence of 0.05% $NaN_3$.

The purified proteoglycan thus obtained exhibits the following characteristics:

1) Molecular mass: 19,000 kD ± 20%.
2) Sedimentation coefficient $S_{20W}$: 58 ± 20%.
3) Stability to enzymes: Not digestible with Chondroitinase A, B, C, Heparinase, Heparitinase, Hyaluronidase and Keratinase.
4) Gelation: Forms gel in aqueous salt solution containing more then 6mM $CaCl_2$ or in deionized water.
5) Shape determined with atomic force microscopy in liquid and electron microscopy: circle of 400-500nm diameter with 10-20 arms 200-300nm long.
6) Stability: circle portion dissociates from arms in aqueous salt solutions containing less then I mM $CaCl_2$ or in the presence of EDTA.
7) $Ca^{2+}$ binding determined by flame ionization spectrometry: binds ca. 7000 moles of $Ca^{2+}$/mole of proteoglycan at 2mM $CaCl_2$ and ca. 70,000 moles of $Ca^{2+}$/mole of proteoglycan at 20mM $CaCl_2$.
8) Dissociation fingerprinting: Dissociation of proteoglycan by 1%SDS at 100°C gave nine fragments ranging from 38 - 1500 kD on a 5-20% linear gradient polyacrylamide gel after electrophoresis. These fragments had apparent molecular masses of ca. 1500 kD, 500 kD, 250 kD, 150 kD, 148 kD, 135 kD, 108 kD, 70 kD, and 38 kD. EDTA and heating at 80°C produced fragments of Mr 1500 x $10^3$, 250 x $10^3$ on gel filtration chromatography. Trypsin digestion produced fragments of Mr 124 x $10^3$, 70 x $10^3$, 27 x $10^3$, 10 x $10^3$ on gel filtration chromatography.

Table I shows approximate amino acid (measured by HPLC pico-tag) and approximate total sugar composition (measured by gas chromatography after methanolysis, reacetylation and silylation) :
This proteoglycan consists of approximatively 36 % by weight proteins and 64 % by weight carbohydrates.

Table I

| Intact proteoglycan (PG) | | | Isolated glycans | |
|---|---|---|---|---|
| $\frac{\text{mol amino acid}}{\text{mol PG}}$ (mol %) | | | $\frac{\text{mol amino acid}}{\text{mol glycan}}$ (mol %) | |
| Asx | 12,736 | 13.4 | 1.2 | 33.4 |
| Thr | 8,196 | 8.6 | 0.6 | 16.7 |
| Ser | 6,179 | 6.7 | 0.3 | 8.4 |
| Glx | 11,475 | 12.0 | 0.7 | 19.5 |
| Pro | 5,611 | 6.0 | 0.0 | 0.0 |

Table I (continued)

| Intact proteoglycan (PG) | | | Isolated glycans | |
|---|---|---|---|---|
| mol amino acid / mol PG (mol %) | | | mol amino acid / mol glycan (mol %) | |
| Gly | 12,484 | 13.1 | 0.5 | 13.8 |
| Ala | 9,205 | 9.7 | 0.1 | 2.8 |
| Val | 5,296 | 5.8 | 0.0 | 0.0 |
| Met | 693 | 0.8 | 0.0 | 0.0 |
| Ile | 3,287 | 4.5 | 0.0 | 0.0 |
| Leu | 6997 | 7.4 | 0.1 | 2.7 |
| Tyr | 3,972 | 4.2 | 0.0 | 0.0 |
| Phe | 3,530 | 3.7 | 0.1 | 2.7 |
| His | 945 | 1.0 | 0.0 | 0.0 |
| Lys | 1,261 | 1.3 | 0.0 | 0.0 |
| Arg | 1,765 | 1.8 | 0.0 | 0.0 |
| **Total** | 94,629 | 100.0 | 3.6 | 100.0 |
| mol carbohydrate / mol PG (mol %) | | | mol carbohydrate / mol glycan (mol %) | |
| Fucose | 15,069 | 33.9 | 9.9 | 34.7 |
| GlcUA | 4,602 | 10.3 | 2.0 | 7.2 |
| Man | 4,602 | 9.1 | 2.7 | 9.6 |
| Gal | 10,907 | 24.5 | 7.4 | 26.1 |
| GlcNAc | 9,836 | 22.2 | 6.3 | 22.3 |
| **Total** | 44,449 | 100.0 | 28.3 | 100.0 |
| | mol /mol PG | | | |
| $SO_4^{--}$ | ≥8,241 | | | |

Standard deviation is less then 20% of each value. Asx signifies Asn or Asp; Glx signifies Glu or Gln. It is also noted that apparant amounts of Ile and Leu are somewhat variable depending on the preparation. The amount of uronic acid determined colormetrically is usually 2 times higher then the amount determined by gas chromatography. $SO_4^{--}$ was determined by HPLC ion chromatography after hydrolysis ofPG.

The N-terminal sequence of the backbone of the molecule is as follows:

**Seq. I** **Pro-Leu-Phe-Thr-Val-Pro-Ile-Tyr-Val-Pro-Glu-Asp-Gln-Leu**

**Seq. II** **Pro-Glu-Val-Gly-Val-Pro-Ile-Tyr-Val-Pro-Glu-Asp-Gln-Leu**

**Seq. III** **Pro-Val-Val-Gly-Val-Pro-Ile-Tyr-Val-Pro-Glu-Asp-Gln-Leu**

preferably Sequence I.

Trypsin digestion of the molecule provides peptides having the sequences:

**Seq. IV** **Phe-Val-Val-Met-Arg**

**Seq. V** **Pro-Gln-Asp-Pro-Phe**

Seq. VI Leu-Ala-Gly-Val-Val-Ile

Seq. VII Pro-Gln-Ala-Ser-Ser-Gly

Seq. VIII Ala-Ala-Gln-Trp-Ile-Gly-Gln-Lys

b. Isolation of acidic glycans from the *Microciona prolifera* proteoglycan

Frozen proteoglycan as obtained above is extracted with water/methanol/chloroform 3/8/4 V/V/V, and the nonlipid fraction was pelleted by centrifugation at 4000 x g for 15 min at +4°C. This extraction is repeated and the pellet is dried under a vacuum. The pellet is wetted in ethanol and resuspended in 0.1 M Tris pH 8, 1mM $CaCl_2$ and 100-200µg Pronase (Calbiochem) (preincubated for 30 min at 60°C in 0.1M Tris pH 8, 1mM $CaCl_2$ per 1-2mg dried powder material), and the pellet is digested at 60°C for three days. Two more equivalent portions of preincubated pronase are added at 24 h intervals. DNAse I is then added (30µg) and incubation is continued at 37°C in the presence of 10mM $MgCl_2$. The digested sample is then treated again with pronase and chromatographed through G-25 Sephadex (Phannacia) column eluted with 10mM pyridine acetate pH 5, void volume fractions are collected and lyophilized, and the glycans thus obtained are dissolved in 50mM NaOH in the presence of 1M $NaHBO_4$ and incubated at 45°C for 16h (NaOH treatment may also be omitted). The glycans are passed through Dowex AG 50W-X8 column in H+ form (Bio-Rad) eluted with water, nonbound glycans are immediately neutralized and electrophoresed on a 5-20% or 10-40% linear polyacrylamide gradient gels (Tris/borate-EDTA), and separated acidic glycans of Mr 200 x $10^3$ are eluted from gels. (Optionally, the acidic glycans can be sepatated by gel filtration rather than electrophoresis). The isolated acidic glycan molecules are desalted using P-2 column (Bio-Rad) eluted with 10mM pyridine acetate pH 5, lyophilized and stored at -20°C.

The acidic glycan fraction is comprised of two major glycans of apparent molecular mass determined by gel electrophoresis using glycosaminoglycan standards of ca. 200 kD and 6 kD. The glycans have the following molar composition (expressed as moles of monosaccharide units / mole of glycan), as determined by gas chromatography, as shown in Table II:

Table II

| | 200 kD glycan | 6 kD glycan |
|---|---|---|
| Fuc | 680 | 3 |
| Man | 20 | 2 |
| Gal | 180 | 5 |
| GlcNAc | 190 | 14 |
| GlcUA | 320 | 7 |
| Asn | 1 | 1 |

Standard deviation is less then 20% of each value. Per mole of proteoglycan, there are 20 moles of the 200 kD glycan and 1000 moles of the 6 kD glycan. The glycans are not digestible with Chondroitinase A, B, C, Heparinase, Heparitinase, Hyaluronidase or Keratinase. They are soluble in aqueous solutions and do not form gels in 6mM $CaCl_2$ salt solutions. At higher concentrations, e.g. > 1 mg/ml water, they will undergo hydrolysis at room temperature.

After partial acid hydrolysis of isolated glycans fragments were purified by ion exchange chromatography and high performance liquid chromatography. Methylation analysis, sequential enzymatic and chemical degradation, $^1$H-NMR spectroscopy, and fast atom bombardment-mass spectrometry of three purified fragments showed following oligosaccharide structures:

**Structure 1**

6
Pyr< >Galβ1-4GlcNAcβ1-3Fuc
4

is repeated 1000 times per mole proteoglycan.

**Structure 2**

GlcNAcβ1-3Fuc
3
|
$SO_3$

is repeated 2000 times per mole proteoglycan.

**Structure 3**

Galα1-2Galβ1-4GlcNAcβ1-3Fuc
3
|
$SO_3$

is repeated 2000 times per mole proteoglycan.

**EXAMPLE 2 : Preparation of proteoglycans and acidic glycans from *Halichondria panicea***

Extraction of proteoglycan from *Halichondria panicea* and isolation of acidic glycans from *Halichondria panicea* proteoglycan is performed as described in example 1 for *Microciona prolifera.* The proteoglycan thus obtained has the following characteristics:

1) Molecular mass: 10,000 kD ± 20%.
2) Sedimentation coefficient of $S_{20W}$ 42 ± 20%.
4) Stability to enzymes: Not digestible with Chondroitinase A, B, C, Heparinase, Heparitinase, Hyaluronidase and Keratinase.
5) Gelation: Forms gel in aqueous salt solution containing more then 6mM $CaCl_2$ or in deionized water.

This proteoglycan consists of approximately 79 % protein and 21 % carbohydrate by weight. It has an approximate amino acid composition (as measured by HPLC pico-tag) and approximate total sugar composition (as measured by gas chromatography) as shown in Table III :

Table III

| Amino acid composition and carbohydrate composition | |
|---|---|
| Intact proteoglycan (PG) | |
| amino acid | mol % |
| Asx | 9.1 |
| Glx | 9.2 |
| Ser | 7.0 |
| Gly | 9.9 |

Table III (continued)

| Amino acid composition and carbohydrate composition | |
|---|---|
| Intact proteoglycan (PG) | |
| amino acid | mol % |
| Arg | 7.6 |
| Thr | 10.2 |
| Ala | 7.0 |
| Pro | 8.2 |
| Tyr | 4.6 |
| Val | 8.6 |
| Met | 2.5 |
| Cys | 0.1 |
| Ile | 6.0 |
| Leu | 5.5 |
| Phe | 4.8 |
| **Total** | 100.0 |
| carbohydrate | (mol %) |
| Fuc | 12.5 |
| Xyl | 1.9 |
| GlcUA | 3.2 |
| GalUA | 1.7 |
| Man | 16.7 |
| Gal | 36.2 |
| Glc | 13.6 |
| GlcNAc | 14.2 |
| **Total** | 100.0 |
| | mol/mol PG |
| $SO_4^{--}$ | ≥6,250 |

Standard deviation is less then 20% of each value. Asx signifies Asn or Asp; Glx signifies Glu or Gln. It is also noted that apparant amounts of Ile and Leu are somewhat variable depending on the preparation. The amount of uronic acid determined colorimetrically is usually 2 times higher then the amount determined by gas chromatography. $SO_4^{--}$ was determined by HPLC ion chromatography after hydrolysis ofPG.

Isolation of acidic glycans from this proteoglycan in the manner described in example 1 gives seven glycans having apparent molecular mass determined by gel electrophoresis using glycosaminoglycan standards of ca. > 1000 kD, 600 kD, 160 kD, 150 kD, 110 kD, 82, kD, and 50 kD.

**EXAMPLE 3 : Preparation of proteoglycans and acidic glycans from *Mycale lingua.***

Extraction of proteoglycan from *Mycale lingua* and isolation of acidic glycans from *Mycale* lingua proteoglycan is performed as described in example 1 for *Microciona prolifera.* The proteoglycan thus obtained has the following characteristics:

1) Molecular mass: 12,000 kD ± 20%.
2) Sedimentation coefficient of $S_{20W}$ 48 ± 20%.
4) Stability to enzymes: Not digestible with Chondroitinase A, B, C, Heparinase, Heparitinase, Hyaluronidase and Keratinase.
5) Gelation: Forms gel in aqueous salt solution containing more then 6mM $CaCl_2$ or in deionized water.

This proteoglycan consists of approximately 58% protein and 42% carbohydrate by weight. It has an approximate amino acid composition (as measured by HPLC pico-tag) and approximate total sugar composition (as measured by gas chromatography) as shown in Table IV :

Table IV

| Amino acid composition and carbohydrate composition | |
|---|---|
| Intact proteoglycan (PG) | |
| amino acid | (mol %) |
| Asx | 10.8 |
| Glx | 9.6 |
| Ser | 6.3 |
| Gly | 7.7 |
| Arg | 9.5 |
| Thr | 10.9 |
| Ala | 8.0 |
| Pro | 7.9 |
| Tyr | 0.5 |
| Val | 9.0 |
| Met | 1.8 |
| Cys | 0.2 |
| Ile | 6.2 |
| Leu | 6.0 |
| Phe | 5.6 |
| **Total** | 100.0 |
| carbohydrate | (mol %) |
| Fuc | 29.7 |
| Xyl | 1.0 |
| GlcUA | 11.5 |
| GalUA | 0.8 |
| Man | 11.0 |
| Gal | 15.3 |
| Glc | 16.7 |
| GalNAc | 6.3 |
| GlcNAc | 7.7 |
| **Total** | 100.0 |
| | mol/mol PG |
| $SO_4^{--}$ | ≥ 12,000 |

Standard deviation is less then 20% of each value. Asx signifies Asn or Asp; Glx signifies Glu or Gln. It is also noted that apparant amounts of Ile and Leu are somewhat variable depending on the preparation. The amount of uronic acid determined colormetrically is usually 2 times higher then the amount determined by gas chromatography. $SO_4^{--}$ was determined by HPLC ion chromatography after hydrolysis ofPG.

**EXAMPLE 4 : Preparation of proteoglycans and acidic glycans from Clionacelata :**

Extraction of two proteoglycans from *Cliona celata* and isolation of acidic glycans from *Cliona* celata proteoglycans is performed as described in example 1 for *Microciona prolifera* with the exception that precipitation with $CaCl_2$ could be omitted. Two proteoglycan designated CPG1 (more abundant in the first extraction) and CPG2 (more abundant in the second extraction) thus obtained has the following characteristics:

1) Molecular mass: CPG1 >20,000 kD ± 20%; CPG2 6,000 kD.
2) Sedimentation coefficient of CPG1 $S_{20W}$ 125 ± 20%; CPG2 26 $S_{20W}$ ± 20%.
4) Stability to enzymes: Not digestible with Chondroitinase A, B, C, Heparinase, Heparitinase, Hyaluronidase and Keratinase.
5) Gelation: Both proteglycans form viscous gels in aqueous salt solution containing more then 6mM $CaCl_2$ or in

deionized water.

CPG1 consists of approximately 26 % protein and 74 % carbohydrate by weight (determined colorimetrically). CPG2 consists of approximately 32 % protein and 68 % carbohydrate by weight. They have an approximate amino acid composition (as measured by HPLC pico-tag) and approximate total sugar composition (as measured by gas chromatography) as shown in Table V :

Table V

| Amino acid composition and carbohydrate composition | | |
|---|---|---|
| Intact proteoglycan (CPG1) | | (CPG2) |
| amino acid (mol %) | | (mol %) |
| Asx | 1.0 | 7.8 |
| Glx | 5.6 | 9.5 |
| Ser | 7.1 | 11.3 |
| Gly | 10.6 | 10.9 |
| Arg | 23.6 | 6.0 |
| Thr | 18.1 | 14.1 |
| Ala | 0.7 | 7.7 |
| Pro | 12.9 | 10.7 |
| Tyr | 8.3 | 0.7 |
| Val | 1.9 | 6.1 |
| Met | 2.4 | 2.4 |
| Cys | 0.3 | 0.2 |
| Ile | 1.0 | 3.9 |
| Leu | 1.3 | 5.1 |
| Phe | 0.8 | 3.6 |
| Lys | 4.3 | 0.1 |
| **Total** | | |
| carbohydrate | | (mol %) |
| Fuc | 11.0 | 17.8 |
| Xyl | 2.2 | 2.2 |
| GlcUA | 9.4 | 11.0 |
| GalUA | 0.7 | 1.1 |
| Man | 1.2 | 5.9 |
| Gal | 6.8 | 12.8 |
| Glc | 17.2 | 18.5 |
| GalNAc | 32.5 | 16.2 |
| GlcNAc | 19.0 | 14.8 |
| **Total** | 100.0 | |
| | mol/mol PG | mol/mol PG |
| $SO_4^{--}$ | ≥ 20,000 | ≥ 6,000 |

Standard deviation is less then 20% of each value. Asx signifies Asn or Asp; Glx signifies Glu or Gln. It is also noted that apparant amounts of Ile and Leu are somewhat variable depending on the preparation. The amount of uronic acid determined colorimetrically is usually 2 times higher then the amount determined by gas chromatography. $SO_4^{--}$ was determined by HPLC ion chromatography after hydrolysis ofPG.

**EXAMPLE 5 : Preparation of acidic glycans from Lytechinus pictus :**

*Lytechinus pictus* sea urchin eggs and/or embryos (from 2 cell stage to plutes stage) were washed with sterile sea water and pelleted embryos were extracted with water/methanol/chloroform 3/8/4 V/V/V, and the nonlipid fraction was pelleted by centrifugation at 4000 x g for 15 min at +4°C. This extraction is repeated and the pellet is dried under

a vacuum. The pellet is wetted in ethanol and resuspended in 0.1 M Tris pH 8, 1mM $CaCl_2$ and 100-200µg Pronase (Calbiochem) (preincubated for 30 min at 60°C in 0.1M Tris pH 8, 1mM $CaCl_2$ per 1-2mg dried powder material), and the pellet is digested at 60°C for three days. Two more equivalent portions of preincubated pronase are added at 24 h intervals. DNAse I is then added (30µg) and incubation is continued at 37°C in the presence of 10mM $MgCl_2$. The digested sample is then treated again with pronase and chromatographed through G-25 Sephadex (Pharmacia) column eluted with 10mM pyridine acetate pH 5, void volume fractions are collected and lyophilized, and the glycans thus obtained are dissolved in 50mM NaOH in the presence of 1M $NaHBO_4$ and incubated at 45°C for 16h (NaOH treatment may also be omitted). The glycans are passed through Dowex AG 50W-X8 column in H+ form (Bio-Rad) eluted with water, nonbound glycans are immediately neutralized and electrophoresed on a 5-20% or 10-40% linear polyacrylamide gradient gels (Tris/borate-EDTA), and separated acidic glycans of Mr 200 x $10^3$ are eluted from gels. (Optionally, the acidic glycans can be sepatated by gel filtration rather than electrophoresis). The isolated acidic glycan molecules are desalted using P-2 column (Bio-Rad) eluted with 10mM pyridine acetate pH 5, lyophilized and purified by affinity chromatography with the Block 2 monoclonal antibodies of ref Misevic et al mentioned above stored at -20°C.

1) Molecular mass: 580 kD ± 20%.
2) Sedimentation coefficient 8.5 $S_{20W}$ ± 20%.
4) Stability to enzymes: Not digestible with Chondroitinase A, B, C, Heparinase, Heparitinase, Hyaluronidase and Keratinase.
5) Gelation: self-interacton-oligomerization in aqueous salt solution containing more then 6mM $CaCl_2$ or in deionized water.

Table VI

| $\frac{\text{mol carbohydrate}}{\text{mol acidic glycan}}$ (mol %) | | |
|---|---|---|
| Fuc | 737 | 25.40 |
| Xyl | 108 | 3.73 |
| Gal | 39 | 1.34 |
| Glc | 12 | 0.41 |
| Uronic acids | 786 | 27.10 |
| CalNAc | 506 | 17.46 |
| GlcNAc | 712 | 24.56 |
| **Total** | 2,900 mol/mol | 100.00 |
| $SO_4^{--}$ | 1.600 | |

Standard deviation is less then 20% of each value. The amount of uronic acid determined colormetrically is usually 2 times higher then the amount determined by gas chromatography. $SO_4^{--}$ was determined by HPLC ion chromatography after hydrolysis of PG.

**EXAMPLE 6 : *Ex vivo* stimulation of human NK cells proliferation by *Microciona prolifera* proteoglycan and by its acidic glycans**

Human peripheral blood mononuclear cell (PBMC) are isolated from 10 ml of blood by centrifugation on Ficoll gradient (Pharmacia). Stimulation of PBMC proliferation with 100 µg/ml acidic glycans or proteoglycans is performed in the presence of complete medium (RPMI 1640, 5% human AB serum, 2mM L-Glutamine, 1mM Na pyruvate, non-essential amino acids and 50µg/ml Kanamycin). After 5 days 5U/ml of human recombinant IL-2 is added . One half of medium is changed when it becomes acidic. After 7, 14, 21, 28 and 35 days cells were analyzed by FACS using antibodies against following markers: CD3, TCR αβ, TCR γδ, CD4, CD8 - T cells; CD16, CD56 - NK cells; CD20 - B cell; CD14 - monocytes. Results from five different donors after 3 weeks: In the PBMC cultures treated with acidic glycans, NK cells population (CD 16 and CD 56 positive) and (CD3, TCR αβ, TCR γδ, CD4, CD8, CD20 and CD14 negative) increased from 1-5 % to 30-80 % of the total PBMC, whereas untreated controls remained at a level of 1-5 % NK cells. Specific stimulation of NK cells proliferation by glycans was confirmed by $^3$H thymidine incorporation only in isolated clones of NK cells and not αβT cells isolated from the same PBMC cultures.

**EXAMPLE 7 : *Ex vivo* stimulation of human NK cells proliferation by *Mycale lingua* and *Cliona celata* proteoglycans and by its acidic glycans was similar to *Microciona prolifera* proteoglycan.**

**EXAMPLE 8 : *Ex vivo* stimulation of human NK cells proliferation by *Lytechinus pictus* acidic glycan with 580 kD was similar to *Microciona prolifera* proteoglycan.**

**EXAMPLE 9 : Stimulation of human γδT cells proliferation (ex *vivo*) by *Microciona prolifera* proteoglycan, *Halichondria panicea* proteoglycan and /or their acidic glycans**

Same culturing procedure as described in the previous example shows that *Microciona prolifera* acidic glycans stimulate only one subpopulation of γδT cells via T cell receptor with an increase from 5% to 20%. *Halichondria panicea* proteoglycan and its acidic glycans stimulate a different population of γδT cells from 5% to 70%. These data are confirmed by $^3H$ thymidine incorporation in isolated clones stimulated by specific acidic glycans.

**EXAMPLE 10 : Anti-tumorogenic and anti-metastatic activity of proteoglycans from *Microciona prolifera* (*in vivo*)**

Seven C-57 black mice are injected i.p. with 300μg proteoglycan from *Microciona prolifera*/200μl 0.2M NaCl, 2mM $CaCl_2$, 20mM Tris pH 7.4/animal, every day for five days. At day five, animals are injected with 2.5 X $10^4$ B-16 melanoma cells per animal. Animals are immunized for five more days with proteoglycan as described above. The appearance of tumor, tumor growth, survival of animals and appearance of metastasis are observed in immunized animals and compared with control animals injected with buffer. Control animals which have not received proteoglycan all exhibit marked melanoma growth followed by metastasis. Compared to controls, treated animals exhibit a 20% delay in the time of appearance and 50% reduction in growth of syngenic B16 melanomas, a 12% increase in the total time of survival of all immunized mice ($p = 0.0044$), and complete inhibition of metastasis.

**EXAMPLE 11 : Anti-tumorogenic and anti-metastatic activity of proteoglycans and their acidic glycans from *Halichondria panicea Mycale lingua, Cliona celata* and *Lytechinus pictus* were similar to to *Microciona prolifera* proteoglycan (*in vivo*).**

**EXAMPLE 12 : Cloning and expression of gene for proteoglycans from *Microciona prolifera***

Proteoglycan (PG) cDNA is isolated from a random-primed cDNA library created using poly(A)$^+$RNA from *Microciona prolifera* cells. This cDNA library is screened using the N-terminal amino acid sequence of PG described in example 1 above by colony hybridization techniques, i.e., expressing the library in an expression system, preferably E. coli, lysing the colonies, e.g., on nitrocellulose filters, denaturing their DNA in situ and fixing it on the filter, hybridizing with labeled, preferably radiolabeled, oligonucleotide probes of at least 30 base pairs having cDNA base sequences corresponding to all or a portion of the N-terminal sequence of PG, identifying hybridized colonies, and retrieving the corresponding vectors from the library, using chromosome walking techniques if necessary to isolate and characterize one or more cDNA fragments containing one or more regions coding for glycosylation sites for N-linked glycans. (Note that the cDNA is repetitive, so it is not necessary to clone, isolate and characterize the entire sequence). Once the desired portion of cDNA has been isolated, it is expressed in a suitable expression system, preferably a eukaryotic system, most preferably a sponge. The PG is isolated from the sponge or from the culture medium of the expression system, e.g., using the procedures outlined above.

## Claims

**1.** A fucose-containing proteoglycan or fucose-containing acidic glycan thereof obtainable from the phylum Porifera and/or of the phylum Echinodermata, and capable of stimulating the proliferation of mammalian NK cells and/or γδ T cells of larger mammals, when it is brought into contact with the cells, for use as a medicament.

**2.** A fucose-containing proteoglycan or acidic glycan thereof for use as a medicament according to claim 1, wherein the fucose containing proteoglycan or acidic glycan thereof capable of being isolated from a marine sponge of the genus Microciona and/or Halichondria and/or Mycale and/or Cliona.

**3.** A fucose-containing proteoglycan or acidic glycan thereof for use as a medicament according to claim 1 capable of being isolated from a sea urchin of the genus Lytechinus.

4. A fucose-containing proteoglycan or acidic glycan thereof for use as a medicament according to claims 1-3, comprising an aciding glycan which is capable of binding to monoclonal antibodies that block the aggregation of sponge cells.

5. Use of a fucose-containing proteoglycan or acidic glycan thereof according to claim 1, in the manufacture of a medicament for treating cancer or viral or retroviral infections.

6. A pharmaceutical composition comprising a fucose-containing proteoglycan or acidic glycan thereof according to claim 1, together with a pharmaceutically acceptable diluent or carrier.

7. A method for ex vivo stimulation of proliferation of mammalian NK cells and/or γδ T cells comprising a fucose-containing proteoglycan or acidic glycan thereof according to claim 1.

8. A method of screening for or detecting an immunosuppressive compound comprising measuring proliferation of NK cells and/or γδ T cells in a system containing an NK cell or γδ T cell stimulatory concentration of a fucose-containing proteoglycan or acidic glycan thereof in the presence and absence of a test compound.

## Patentansprüche

1. Ein Fucose Enthaltendes Protoglycan oder ihr säuerliches Fucose Enthaltendes Glycan gewinnbar von der Phylum *Porifea* und/oder von der phylum *Echinodermata,* und dazu fähig die Proliferation von menschlichen NK zelten und/oder yö T Zellen von grösseren Säugetieren zu stimulieren, wenn es in Kontakt mit den Zellen gebracht wird, für die Verwendung eines Medikamentes.

2. Ein Fucose Enthaltendes Protoglycan oder ihr säuerliches Glycan, für die Verwendung als Medikament übereinstimmend mit dem Anspruch 1, worin Fucose enthaltende Protoglycan oder ihr säuerliches Glycan enthalten ist, welche von einem Marinen Schwamm vom Genus *Microiona* und/oder *Halichondria* und/oder *Mycale* und/oder *Cliona* isolierbar ist.

3. Ein Fucose Enthaltendes Protoglycan oder ihr säuerliches Glycan, für die Verwendung als Medikament, übereinstimmend mit dem Anspruch 1, welches von einem Seeigel vom dem Genus *Lytechinus* isolierbar ist.

4. Ein Fucose Enthaltendes Protoglycan oder ihr säuerliches Glycan, für die Verwendung als Medikament übereinstimmend mit dem Ansprüchen 1-3, enthalten ein säuerliches Glycan welches fähig ist an monocloalen Antikörpern zu binden, welche die Aggregation von Schwamm Zellen blockieren.

5. Benutzung einer Fucose Enthaltenden Protoglycane oder ihr säuerliches Glycane welche übereinstimmend mit dem Anspruch 1 ist, in der Manufaktur von einem Medikament für die Bekämpfung von Krebs oder retroviralen Infektionen.

6. Eine Pharmazeutische Komposition, welche ein Fucose enthaltende Protoglycane oder ihr säuerliches Glycane enthaltet, welche übereinstimmend mit dem Anspruch 1, zusammen mit einer pharmazeutischen akzeptablen Diluenten oder Träger.

7. Eine Methode der *ex vivo* Stimulation von Proliferation, von Säugetier NK Zellen und/oder yö T Zellen, enthaltend eine Fucose enthaltende Protoglycan oder ihr säuerliches Glycan welche übereinstimmt mit dem Anspruch 1.

8. Eine Methode des Screening, für die Entdeckung von immunosuppresive Substanzen, enthaltend Messungen von Proliferation von NK Zellen und/oder yö T Zellen in einem System mit einer Stimulierender Konzentation von Fucose enthaltenden Protoglycan oder ihr säuerliches Glycan, in Präsenz oder Absenz von einer getesteten Substanz, auf die enthaltenen NK Zellen oder yö T Zellen.

## Revendications

1. Protéoglycane comprenant du fucose ou glycane acide comprenant du fucose et provenant dudit protéoglycane, susceptible d'être obtenu à partir de phylum Porifera et/ou de phylum Echinodermata et apte à stimuler la prolifération de cellules NK de mammifères et/ou de cellules γδ T de plus gros mammifères, lorsqu'il est amené en contact avec ces cellules, pour une utilisation en tant que médicament.

2. Protéoglycane comprenant du fucose ou glycane acide comprenant du fucose et provenant dudit protéoglycane pour une utilisation en tant que médicament, selon la revendication 1, dans lequel le protéoglycane comprenant du fucose ou le glycane acide comprenant du fucose et provenant dudit protéoglycane, est susceptible d'être obtenu à partir d'une éponge marine du genre Microciona et/ou Halichondria et/ou Mycale et/ou Cliona.

3. Protéoglycane comprenant du fucose ou glycane acide comprenant du fucose et provenant dudit protéoglycane pour une utilisation en tant que médicament, selon la revendication 1, susceptible d'être obtenu à partir d'un oursin du genre Lytechinus.

4. Protéoglycane comprenant du fucose ou glycane acide comprenant du fucose et provenant dudit protéoglycane pour une utilisation en tant que médicament, selon les revendications 1 à 3, comprenant un glycane acide qui est apte à se lier à des anticorps monoclonaux qui bloquent l'agrégation des cellules d'éponge.

5. Utilisation d'un protéoglycane comprenant du fucose ou d'un glycane acide comprenant du fucose et provenant dudit protéoglycane selon la revendication 1, pour la fabrication d'un médicament pour traiter le cancer ou des infections virales or rétrovirales.

6. Composition pharmaceutique comprenant un protéoglycane comprenant du fucose ou d'un glycane acide comprenant du fucose et provenant dudit protéoglycane selon la revendication 1 et un diluant ou un support pharmaceutiquement acceptable.

7. Procédé de stimulation ex vivo de la prolifération de cellules NK de mammifères et/ou de cellules γδ T, faisant intervenir un protéoglycane comprenant du fucose ou un glycane acide comprenant du fucose et provenant dudit protéoglycane selon la revendication 1.

8. Procédé de criblage ou de détection d'un composé immunodépresseur comprenant la mesure de la prolifération de cellules NK et/ou de cellules γδ T dans un système contenant un protéoglycane comprenant du fucose ou un glycane acide comprenant du fucose et provenant dudit protéoglycane, dans une concentration de stimulation de cellules NK et/ou de cellules γδ T, en présence et en l'absence d'un composé d'essai.